# FASCICULE DE BREVET EUROPEEN

(11) **EP 0 821 697 B1**
(45) Date de publication et mention de la délivrance du brevet: **26.07.2000**
(21) Numéro de dépôt: 96913594.6
(22) Date de dépôt: 16.04.1996
(51) Int. Cl.: C07K 7/06

(54) **PROCEDE DE PREPARATION DE STREPTOGRAMINES**
VERFAHREN ZUR HERSTELLUNG VON STREPTOGRAMINEN
METHOD FOR PREPARING STREPTOGRAMINES

(30) Priorité: 18.04.1995 FR 9504585
(43) Date de publication de la demande: 04.02.1998
(73) Titulaire: Aventis Pharma S.A., 92160 Antony (FR)
(72) Inventeur: BARRIERE, Jean-Claude, F-91440 Bures-sur-Yvette (FR); GRONDARD, Luc, F-91080 Courcouronnes (FR); LEFEVRE, Patrick, F-94300 Vincennes (FR); MUTTI, Stéphane, F-92600 Asnières (FR)
(86) Numéro de dépôt international: FR9600575
(87) Numéro de publication internationale: WO9633213

(56) Documents cités:
- EP-A- 0 614 910
- FR-A- 2 689 518
- TETRAHEDRON LETT. (1983), 24(34), 3631-4 CODEN: TELEAY;ISSN: 0040-4039, XP002012279 TAKATA, TOSHIKAZU ET AL: "Mild and selective oxygen atom transfer: tetrabutylammonium periodate (Bu4NIO4) with metalloporphyrins"

## Description

La présente invention concerne un nouveau procédé de préparation de streptogramines de formule générale : 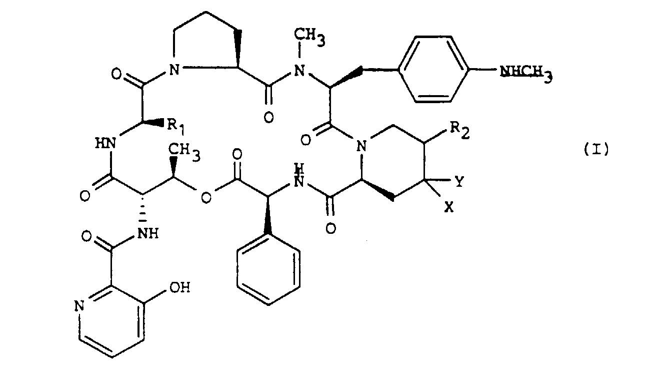 dans laquelle :
- soit le radical R₁ représente un groupe méthyle ou éthyle, le radical R₂ représente un atome d'hydrogène et X et Y forment ensemble un radical oxo,
- soit R₁ représente un radical éthyle, R₂ et X représentent un atome d'hydrogène et Y représente un atome d'hydrogène ou un radical hydroxy,
- soit R₁ représente un radical éthyle R₂ représente un radical hydroxy et X et Y forment ensemble un radical oxo,
à partir d'un dérivé de synergistine de formule générale : 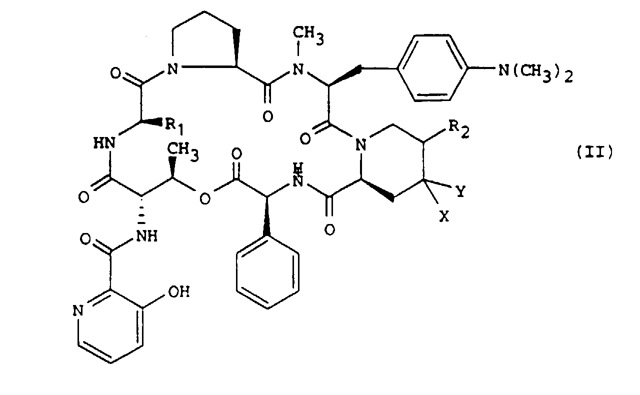 dans laquelle les radicaux R₁, R₂, X et Y sont définis comme ci-dessus.

Les streptogramines sont une classe de composés connue comprenant des composantes du groupe B [auquel appartient les produits de formule générale (I)] qui associés à des composants du groupe A, provoquent une synergie de l'action antimicrobienne.

Le produit de formule générale (I) pour lequel R₁ est éthyle et R₂ est hydrogène est connu sous le nom de pristinamycine IB. Le produit de formule générale (I) pour lequel R₁ est méthyle et R₂ est hydrogène est connu sous le nom de vernamycine Bδ. Le produit de formule générale (II) pour lequel R₁ est éthyle et R₂ est hydrogène est connu sous le nom de pristinamycine IA. Le produit de formule générale (II) pour lequel R₁ est méthyle et R₂ est hydrogène est connu sous le nom de pristinamycine IC ou vernamycine Bγ. Le produit de formule générale (II) pour lequel R₁ est éthyle et R₂ est hydroxy est connu sous le nom de pristinamycine ID.

Des méthodes générales de déméthylation étaient déjà connues comme par exemple les méthodes décrites dans Tet. Lett., 18, 1567 (1977) ; J. Org. Chem., 49, 2795 (1984) ; J. C. S. Chem. Comm., 905 (1989), Tet. Lett., 33, 6991 (1992), cependant ces méthodes n'étaient pas adaptables à des produits fragiles comme les streptogramines, soit parce que la réaction n'intervenait pas, soit parce que les conditions opératoires étaient dégradantes vis à vis de ces produits. D'autres encore faisaient intervenir des réactifs toxiques et non totalement éliminables du produit final, ce qui est inacceptable du point de vue pharmaceutique.

Il a maintenant été trouvé, et c'est ce qui fait l'objet de la présente invention, que les streptogramines de formule générale (I) pouvaient être obtenues par déméthylation du dérivé correspondant de formule générale (II) par traitement par un periodate en milieu acétique suivi d'un traitement en milieu acide aqueux ou d'un traitement par un agent capable de consommer le formaldéhyde in situ.

Le periodate utilisé est avantageusement le periodate de tétra N-butyl ammonium ou un periodate alcalin (periodate de sodium). La réaction s'effectue dans un solvant tel qu'un solvant chloré (dichlorométhane, chloroforme, dichloréthane, trichloréthane par exemple), un ester (acétate d'éthyle par exemple), un nitrile (acétonitrile par exemple) ou dans le tétrahydrofuranne, la N-méthyl pyrrolidone ou éventuellement un mélange de ces solvants en présence ou non d'éthylène glycol. La réaction s'effectue à une température comprise entre 20 et 40°C.

Le traitement subséquent est une hydrolyse en milieu aqueux libérant du formaldéhyde. Il est possible d'opérer par traitement du produit obtenu, en milieu aqueux homogène additionné d'un acide fort ou, directement en milieu biphasique acide ou non ; notamment on peut opérer dans un mélange dichlorométhane/eau. Dans ce cas de préférence le pH du milieu aqueux sera faiblement acide ; il est entendu que l'acidité du milieu sera apportée indifféremment par addition d'un acide fort ou faible.

Les acides utilisés peuvent être notamment choisis parmi l'acide trifluoracétique, l'acide sulfurique, l'acide chlorhydrique, l'acide méthane sulfonique, l'acide p.toluène sulfonique ou l'acide formique. Le traitement en milieu acide s'effectue à une température comprise entre 0 et 40°C.

Lorsque l'on effectue le traitement subséquent, il est également possible d'additionner en plus un agent capable de consommer le formaldéhyde in situ, cet agent est choisi avantageusement parmi l'hydroxylamine, un bisulfite (bisulfite de sodium par exemple), ou l'eau oxygénée en milieu aqueux. L'opération s'effectue de préférence en milieu biphasique à une température comprise entre 0 et 40°C, à un pH compris entre 1 et 7.

Les produits de formule générale (I) ainsi obtenus peuvent être purifiés, le cas échéant, par les méthodes habituelles comme la cristallisation, la précipitation, la chromatographie flash ou la CLHP.

Les produits de formule générale (I) dans laquelle R₁ représente un radical éthyle, R₂ et X représentent un atome d'hydrogène et Y représente un atome d'hydrogène ou un radical hydroxy sont des produits nouveaux de la famille des streptogramines.

Les exemples suivants donnés à titre non limitatif, illustrent le procédé selon l'invention.

### Exemple 1

On place dans un tricol, 540 g de pristinamycine I brute [pristinamycine I_{A} 72,2 % (433 g), pristinamycine I_{B} 4,2 % (25 g), pristinamycine I_{C} 2,67 % (16 g), pristinamycine I_{D} 3,17 % (19 g)] en solution dans un mélange de 1460 cm³ de dichlorométhane, 500 cm³ d'acide acétique et de 40 cm³ d'éthylène glycol. On ajoute 97,5 g de périodate de tétra N-butyl ammonium en maintenant la température à 30°C. Après 3 heures d'agitation à 30°C, la réaction est arrêtée par addition, sous agitation, de 2000 cm³ d'eau déminéralisée. La phase aqueuse est décantée et la phase organique lavée à nouveau par 2000 cm³ d'eau déminéralisée. La phase aqueuse est décantée et la phase organique concentrée jusqu'à un volume de 800 cm³. On ajoute au concentrat 1000 cm³ de méthyl éthyl cétone et le mélange est concentré sous pression réduite (1,5 kPa) à un volume de 1300 cm³. On ajoute de la méthyl éthyl cétone jusqu'à un volume total de 2400 cm³ et le mélange est refroidi à 0°C. Le solide précipité est filtré, lavé par 3 fois 250 cm³ de méthyl éthyl cétone puis séché à 40°C sous pression réduite (1,5 kPa). On obtient ainsi 441 g d'un solide blanc qui est mis en solution dans 8800 cm³ d'acide chlorhydrique 0,25 N et agité pendant 1 heure puis extrait par 3500 cm³ de dichlorométhane en ajustant le pH de la phase aqueuse à 4 avec de la soude à 30 %. La phase organique est décantée, lavée par 3500 cm³ d'eau puis concentrée à sec sous pression réduite (50 kPa à 30°C) jusqu'à un volume de 1100 cm³ environ. A cette solution on ajoute 2200 cm³ d'éthanol et on poursuit l'évaporation sous pression réduite jusqu'à 1800 cm³. On ajoute alors 3500 cm³ d'éthanol. Les cristaux obtenus sont filtrés à 10°C, filtrés puis rincés par 3 fois 330 cm³ d'éthanol froid, puis séchés à 40°C sous pression réduite (1,5 kPa). On obtient ainsi 360 g de pristinamycine I_{B} sous forme de cristaux blancs, pure à 80,7 %, soit contenant 290,4 g de pristinamycine I_{B}.

Par ailleurs il a été obtenu 1,1 % de vernamycine Bδ soit un rendement de transformation de 41,2 %, et 2% de pristinamycine de formule générale (I) dans laquelle R₁ est éthyle et R₂ est hydroxy soit un rendement de transformation de 63 % (dosage CLHP).

### Exemple 2

On place dans un tricol, 20 g de pristinamycine I [pristinamycine I_{A} 76,5 % (15,3 g), pristinamycine I_{B} 7 % (1,4 g)] en solution dans un mélange de 28 cm³ de dichloro-1,2-éthane, 70 cm³ d'acide acétique et de 2 cm³ d'éthylène glycol. On ajoute 4,9 g de périodate de sodium en maintenant la température à 25°C. Après 6 heures d'agitation, la réaction est arrêtée par addition, sous agitation, de 100 cm³ d'eau déminéralisée. La phase aqueuse est décantée et la phase organique lavée à nouveau par 50 cm³ d'eau déminéralisée. La phase aqueuse est décantée et la phase organique concentrée à sec sous pression réduite. Le solide est repris par 400 cm³ de méthyl isobutyl cétone et le produit extrait par 2 fois 320 cm³ puis 80 cm³ d'acide sulfurique 0,2 N. Les phases aqueuses sont réunies puis extraites par 400 cm³ de dichlorométhane. La phase organique est décantée, concentrée à sec sous pression réduite (30 kPa) à 30°C puis séchée sous pression réduite (150 Pa) à 40°C pour donner 12,5 g d'un solide blanc contenant 72 % (9 g) de pristinamycine I_{B} et 5,6 % (0,7 g) de pristinamycine I_{A}. Rendement de la conversion 84,9 %.

### Exemple 3

On place dans un tricol, 540 g de pristinamycine I brute (pristinamycine I_{A} 433 g, pristinamycine I_{B} 25 g, pristinamycine I_{C} 16 g, pristinamycine I_{D} 19 g) en solution dans un mélange de 1460 cm³ de dichlorométhane, 500 cm³ d'acide acétique et de 40 cm³ d'éthylène glycol. On ajoute 97,5 g de périodate de tétra N-butyl ammonium en maintenant la température à 30°C. Après 3 heures d'agitation à 30°C, la réaction est arrétée par addition sous agitation de 2000 cm³ d'eau déminéralisée contenant 34,7 g de chlorhydrate d'hydroxylamine. La phase aqueuse est décantée puis séparée. La phase organique est lavée par 2000 cm³ d'eau. Après décantation et séparation, la phase organique est concentrée jusqu'à un miel. On verse sur ce concentrat 2500 cm³ d'acétate d'éthyle, puis on concentre jusqu'à un volume final de 1300 cm³. On filtre la suspension à 5°C. Les cristaux sont lavés par 3 fois 400 cm³ d'acétate d'éthyle frais et séchés à 40°C sous 1500 Pa de pression résiduelle. On obtient ainsi, 331 g de produit blanc titrant 91% en pristinamycine IB.

### Exemple 4

On place dans un tricol 180 g de pristinamycine I brute (contenant 111,1 g de pristinamycine IA et 35,6 g de pristinamycine IB) en solution dans un mélange de 444 cm³ de dichlorométhane, 128 cm³ d'acide acétique et de 10 cm³ d'éthylène glycol. On ajoute 25,9 g de periodate de tétra N-butyl ammonium. Après quatre heures d'agitation à 32°C, la réaction est arrêtée par addition, sous agitation, de 1100 cm³ d'eau de ville. Les deux phases sont décantées et séparées. La phase organique est lavée à nouveau 4 fois de suite par, à chaque fois, 1400 cm³ d'eau de ville. Le pH de ces quatre lavages est réajusté à la baisse par 5 ml d'acide chlorhydrique normal pour des décantations faciles. Ces quatre lavages, décantations et séparations sont effectués à 35°C. La phase organique est concentrée d'un facteur deux environ. On verse progressivement sur ce concentrat 600 cm³ d'acétate d'éthyle en amorçant la cristallisation après un tiers d'addition environ. Après l'addition, on poursuit l'alimentaion en acétate d'éthyle avec distillation en parallèle de façon à rester à volume constant dans le ballon, soit environ 600 cm³. Après distillation à volume constant d'environ 800 cm³, la suspension est refroidie à 0°C et filtrée. Le gâteau est lavée par deux fois 125 cm³ d'acétate d'éthyle à 0°C et séché sous pression réduite (1,5 kPa) à 40°C jusqu'à poids constant. On obtient 120 g d'un produit beige clair contenant 110 g de pristinamycine IB.

## Revendications

1. Un procédé de préparation de streptogramines de formule générale : 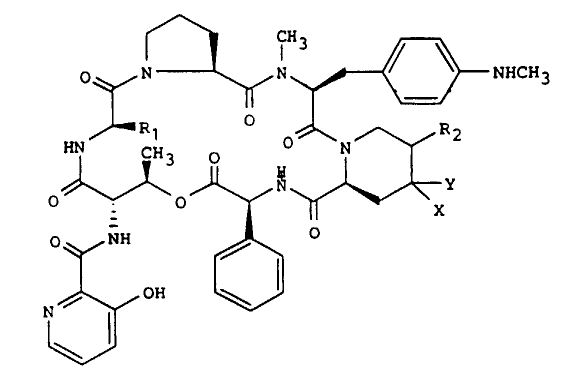 dans laquelle :
- soit le radical R₁ représente un groupe méthyle ou éthyle, le radical R₂ représente un atome d'hydrogène et X et Y forment ensemble un radical oxo,
- soit R₁ représente un radical éthyle, R₂ et X représentent un atome d'hydrogène et Y représente un atome d'hydrogène ou un radical hydroxy,
- soit R₁ représente un radical éthyle R₂ représente un radical hydroxy et X et Y forment ensemble un radical oxo,
par déméthylation d'un dérivé de synergistine de formule générale : 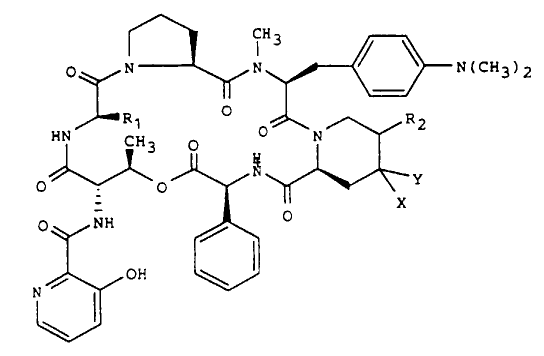 dans laquelle les radicaux R₁, R₂, X et Y sont définis comme ci-dessus, par traitement par un periodate en milieu acétique suivi d'un traitement en milieu aqueux.

2. Procédé selon la revendication 1, caractérisé en ce que le periodate est choisi parmi le periodate de tétra N-butyl ammonium ou un periodate alcalin.

3. Procédé selon la revendication 2, caractérisé en ce que le periodate alcalin est le periodate de sodium.

4. Procédé selon la revendication 1, caractérisé en ce que le traitement subséquent est une hydrolyse en milieu aqueux, soit en milieu homogène additionné d'un acide fort, soit en milieu biphasique acide ou non.

5. Procédé selon la revendication 1, caractérisé en ce que l'on additionne un agent capable de consommer le formaldéhyde in situ, choisi parmi l'hydroxylamine, un bisulfite ou l'eau oxygénée, au cours du traitement subséquent.

6. Un dérivé de streptogramine caractérisé en ce qu'il répond à la formule générale : 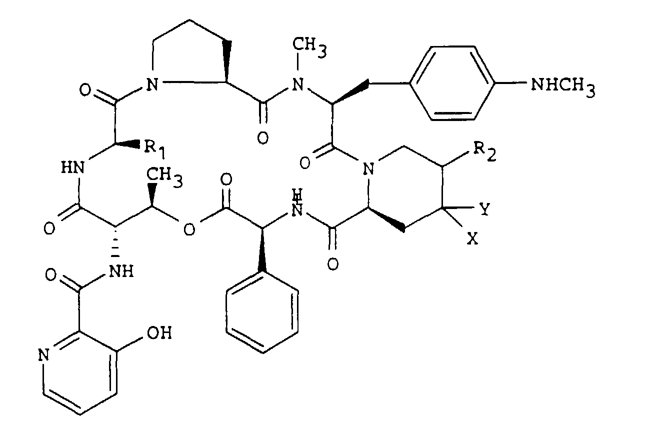 dans laquelle R₁ représente un radical éthyle, R₂ et X représentent un atome d'hydrogène et Y représente un atome d'hydrogène ou un radical hydroxy.

## Patentansprüche

1. Verfahren zur Herstellung von Streptograminen der allgemeinen Formel 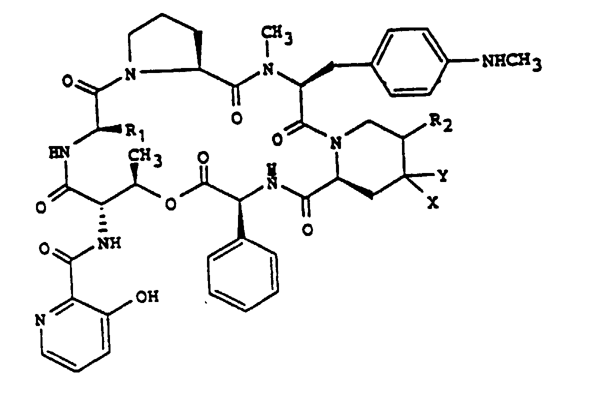 worin
- entweder der Rest R₁ eine Methyl- oder Ethylgruppe bezeichnet, der Rest R₂ ein Wasserstoffatom bezeichnet und X und Y zusammen eine Oxogruppierung bilden,
- oder R₁ eine Ethylgruppe bezeichnet, R₂ und X ein Wasserstoffatom bezeichnen und Y ein Wasserstoffatom oder eine Hydroxylgruppe bezeichnet,
- oder R₁ eine Ethylgruppe bezeichnet, R₂ eine Hydroxygruppe bezeichnet und X und Y zusammen eine Oxogruppierung bilden,
durch Demethylierung eines Derivats von Synergistin der allgemeinen Formel 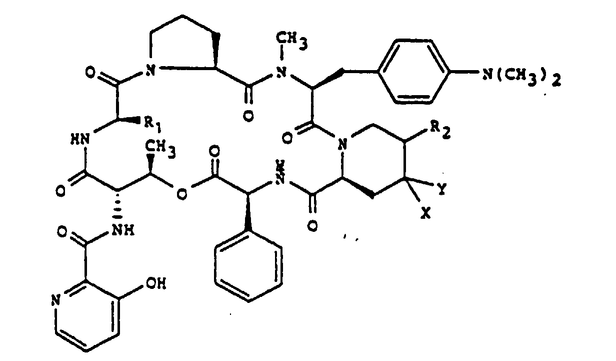 worin die Reste R₁, R₂, X und Y wie vorstehend definiert sind, durch Behandlung mit einem Periodat in einem essigsauren Medium, gefolgt von einer Behandlung in einem wässerigen Medium.

2. Verfahren nach Anspruch 1, dadurch gekennzeichnet, dass das Periodat aus Tetra-N-butylammoniumperiodat oder einem Alkaliperiodat ausgewählt wird.

3. Verfahren nach Anspruch 2, dadurch gekennzeichnet, dass das Alkaliperiodat Natriumperiodat ist.

4. Verfahren nach Anspruch 1, dadurch gekennzeichnet, dass die anschliessende Behandlung eine Hydrolyse in wässerigem Medium, entweder in homogenem Medium unter Zusatz einer starken Säure oder in einem biphasischen sauren oder nicht sauren Medium, ist.

5. Verfahren nach Anspruch 1, dadurch gekennzeichnet, dass man im Verlauf der anschliessenden Behandlung ein Mittel zusetzt, das Formaldehyd in situ verbrauchen kann, ausgewählt aus Hydroxylamin, einem Bisulfit oder oxygeniertem Wasser.

6. Streptograminderivat, dadurch gekennzeichnet, dass es die allgemeine Formel 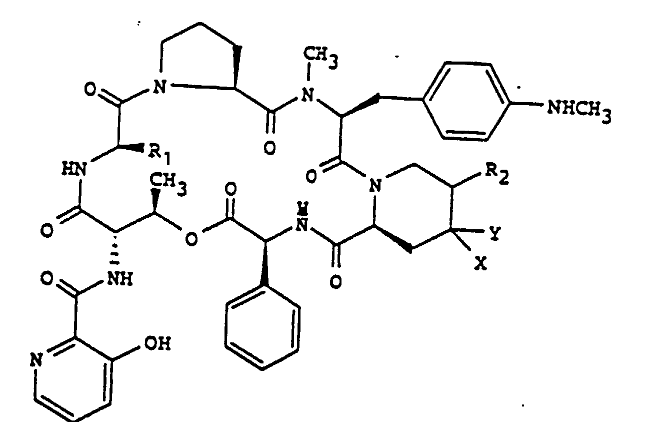 besitzt, worin R₁ eine Ethylgruppe bezeichnet, R₂ und X ein Wasserstoffatom bezeichnen und Y ein Wasserstoffatom oder eine Hydroxylgruppe bezeichnet.

## Claims

1. A process for the preparation of streptogramins of general formula: 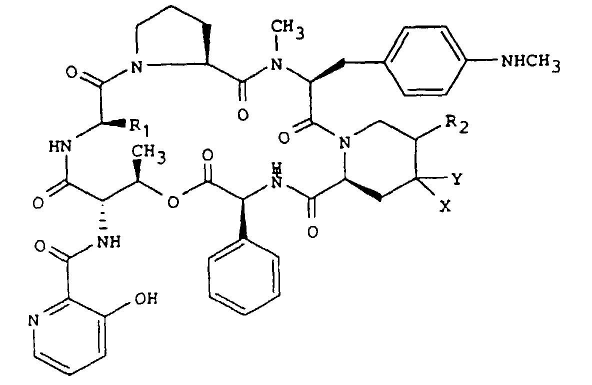 in which:
- either the radical R₁ represents a methyl or ethyl group, the radical R₂ represents a hydrogen atom and X and Y together form an oxo radical,
- or R₁ represents an ethyl radical, R₂ and X represent a hydrogen atom and Y represents a hydrogen atom or a hydroxyl radical,
- or R₁ represents an ethyl radical, R₂ represents a hydroxyl radical and X and Y together form an oxo radical,
by demethylation of a synergistin derivative of general formula: 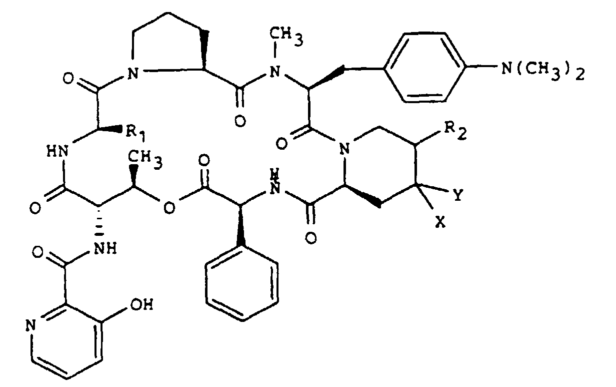 in which the radicals R₁, R₂, X and Y are as defined above, by treatment with a periodate in acetic medium followed by a treatment in aqueous medium.

2. Process according to claim 1, characterized in that the periodate is chosen from tetra-n-butylammonium periodate or an alkaline periodate.

3. Process according to claim 2, characterized in that the alkaline periodate is sodium periodate.

4. Process according to claim 1, characterized in that the subsequent treatment is a hydrolysis, in aqueous medium, either in a homogeneous medium to which is added a strong acid, or in an acidic or non-acidic two-phase medium.

5. Process according to claim 1, characterized in that an agent capable of consuming the formaldehyde in situ is added, this agent being chosen from hydroxylamine, a bisulphite or hydrogen peroxide, during the subsequent treatment.

6. A streptogramin derivative, characterized in that it corresponds to the general formula: 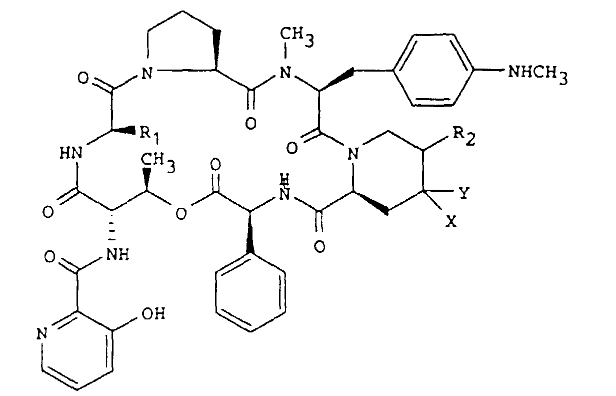 in which R₁ represents an ethyl radical, R₂ and X represent a hydrogen atom and Y represents a hydrogen atom or a hydroxyl radical.
